# EUROPEAN PATENT SPECIFICATION

(11) **EP 3 403 097 B1**
(45) Date of publication and mention of the grant of the patent: **03.07.2019**
(21) Application number: 17700364.7
(22) Date of filing: 13.01.2017
(51) Int. Cl.: G01N 33/558

(54) **METHOD FOR DETECTING AN ANALYTE**
VERFAHREN ZUM ERKENNEN EINES ANALYTEN
PROCÉDÉ POUR LA DÉTECTION D'UN ANALYTE

(30) Priority: 15.01.2016 WO PCT/EP2016/151570
(43) Date of publication of application: 21.11.2018
(73) Proprietor: DSM IP Assets B.V., 6411 TE Heerlen (NL)
(72) Inventor: JAGESAR, Dhiredj Chandre, 6100 AA Echt (NL)
(74) Representative: DSM Intellectual Property
(86) International application number: PCT/EP2017/050662
(87) International publication number: WO 2017/121848

(56) References cited:
- EP-A1- 2 693 213
- WO-A1-02/01229
- WO-A1-2013/037885
- WO-A2-2005/098439
- WO-A2-2008/073222
- WO-A2-2011/160015
- US-A- 3 378 346
- US-A1- 2003 003 522
- US-B2- 8 815 609

## Description

### Field of the invention

The present invention discloses a method for detecting an analyte in a liquid composition, a device, and a kit.

### Background of the invention

Antibiotics are used for combating infectious diseases, both in humans and in animals. It is well-known that misuse of antibiotics such as administration of antibiotics whenever this is not required from a medical point of view or incomplete courses of treatment is the most important cause of the development of antibiotic resistance. Thus, methods for detecting the presence of antibiotics in samples such as e.g. milk, blood, fish, feed, meat, serum, urine, water and the like are of utmost importance in the prevention of the unwanted spread of antibiotics. In many areas, this process of detection can only be performed adequately, provided that a fast and simple test is available.

In general, there are two types of tests suitable for routinely monitoring the presence of antibiotics in samples. Firstly, there are microbial inhibition tests, wherein a test microorganism is contacted with the sample to be tested and the growth (or inhibition of growth) of the microorganism is observed, for instance with the use of an indicator. An example of such a test is described in EP 0 755 456 B1. The major drawback of microbial inhibition tests is that it takes a relatively long time to obtain results.

Secondly, there are competitive immunoassays, wherein the antibiotic to be tested and a reference antibiotic present in the test compete for binding with binding proteins and/or antibodies with affinity for the antibiotics. Visualization is usually done by means of labeling. Examples of such a test are described in WO 2013/037885 and EP 0 593 112 B1. Although these types of tests are in general faster than microbial inhibition tests, they still require extensive handling by the end user and are therefore not user friendly. Also, prior art competitive immunoassays are often not sufficiently sensitive.

In view of the above, there exists considerable room for improvement in the area of antibiotic testing, particularly where it concerns sensitivity.

### Description of the invention

Throughout the present specification and the accompanying claims, the words "comprise", "include" and "having" and variations such as "comprises", "comprising", "includes" and "including" are to be interpreted inclusively. That is, these words are intended to convey the possible inclusion of other elements or integers not specifically recited, where the context allows.

The articles "a" and "an" are used herein to refer to one or to more than one (*i.e.* to one or at least one) of the grammatical object of the article. By way of example, "an analyte may mean one analyte or two or more analytes and 'a receptor' may include two or more receptors.

In a first aspect the invention provides a method for detecting an analyte in a liquid composition, said method comprising:
a) providing a test device having a proximal and a distal end, said test device configured to allow lateral flow from the proximal to the distal end, said test device comprising a solid support comprising the following regions in sequence from the proximal to the distal end:
   i. a sample receiving region,
   ii. a conjugate region comprising a labeled receptor capable to bind the analyte and a labeled control reagent,
   iii. a detection region comprising at least two zones:
      A. a detection zone comprising an immobilized binding agent capable of binding the labeled receptor, when said labeled receptor is unbound by analyte from the sample, and
      B. a control zone comprising an immobilized binding agent capable of binding the labeled control reagent,
   iv. an absorbing region, and
   v. optionally a handling region,
b) contacting the liquid composition with the sample receiving region of the test device,
c) allowing said liquid composition to move from the sample receiving region through the conjugate region and the detection region to the absorbing region, so as to allow said liquid composition comprising the labeled receptor and the labeled control reagent to contact the detection zone and the control zone,
d) detecting a signal at the detection zone and a signal at the control zone, wherein
   i. the absence of analyte in the sample is indicated by the presence of a signal at the detection zone that is more intense than the signal at the control zone, and
   ii. the presence of analyte in the sample is indicated by the absence of a signal at the detection zone or the presence of a signal at the detection zone that is less intense than the signal at the control zone,
wherein the section of the device between the proximal end and the detection region comprises a compound selected from the list consisting of methylcellulose, carboxymethylcellulose, hydroxymethylcellulose, carboxyethylcellulose, and hydroxyl-ethylcellulose.

In the method of the invention a liquid composition is contacted with the sample receiving region of the test device. This can be done for example by dipping the device in the liquid composition. The liquid composition can be conveniently put in a container such as a tube or vial, such that the sample receiving region of the device can be dipped in the liquid.

Before the liquid composition is contacted with the sample receiving region, said liquid composition can be contacted with a compound such as an organic buffer such as a Tris or phosphate buffer, a surfactant such as Tween-20 or Triton X-100 (preferably in a concentration of between 0 and 2% w/v), a protein such as bovine serum albumin, a polyol such as glycerin, and a sugar e.g. a disaccharide such as saccharose.

In an embodiment the method comprises, prior to contacting the liquid composition with the sample receiving region of the test device, adding said liquid composition to a container comprising a buffer such as an organic buffer. The amount of liquid composition added to the container may be between 50 µL and 10 mL, preferably between 50 and 1000 mL, more preferably between 125 and 250 µL. After the sample is contacted with the buffer, the obtained liquid composition may be shaken. Shaking is generally done for 1 to 60 seconds, preferably 1 to 20 seconds, preferably 15 to 45 seconds with about 30 seconds being preferred. Containers that can be used in the present invention may be tubes of any shape and size and from any suitable material available. Containers may also be the wells such as those incorporated in microtiter plates. Using a container facilitates the contacting. For example, the device can be simply dipped into the container with the liquid composition.

The amount of liquid composition added to the sample receiving section is preferably between 50 and 1000 µL, preferably between 75 and 750 µL, more preferably between 100 and 500 µL, in particular between 125 and 250 µL.

The method is suitable for analyzing the presence of an analyte in a sample. A sample may be solid or liquid. When a sample is liquid, it can be contacted with the sample receiving region of the test device as such. When a sample is solid, the analyte(s) needs to be extracted from the sample, which extraction results in a liquid composition, which can be contacted with the sample receiving region of the test device. Methods for extracting liquids from samples depend on the type of sample. Suitable extraction methods for different types of samples are known to the person skilled in the art and include disintegration of the solid sample by homogenization, vortexing with beads, grinding or sonication and/or solvent extraction.

The liquid composition may be derived from a body liquid, an organ, meat or eggs. Analytes may also be present in food products in which these animal products are added as an ingredient. Examples of food products are milk; honey; meat of cow, pig, poultry and fish; sea food such as shrimps; processed meat products such as sausages; ready-to-eat meals; feed; and baby food. Analytes may also be present in body liquids or animal tissues, which are suitable for examination by for example food inspection authorities. Examples are blood, liver tissue, muscle tissue, heart tissue, kidney tissue or pre-urine obtained from the kidney and urine. Urine and blood are suitable for examination prior to slaughtering of an animal. Analytes may also be present in water such as waste water, for example from antibiotics-producing plants or from hospital streams. In a preferred embodiment the liquid composition is milk. Milk may be from cattle (e.g. cows), horses, sheep, goats, yaks, water buffalo, humans, donkeys, reindeers, bison and camels. Analytes may also be present in semi-processed or processed food such as pasteurized products, UHT-products, skimmed or partially skimmed milk, whey, fresh or ripened cheese, yoghurt, cream, butter, sour cream, buttermilk, to name just a few.

In an embodiment the test device is contacted with the liquid composition for 1 to 10 minutes, preferably 1.5 to 4 minutes, more preferably 2 to 3 minutes at a temperature of 40 to 70°C, preferably a temperature of between 50 and 65°C, more preferably a temperature of between 60 and 64°C. Incubation can be carried out with the aid of a thermostatic device such as a water bath or an incubator. In a preferred embodiment the temperature before and after the liquid composition is contacted with the test device is identical. Incubation may be stopped as soon as a signal is detected at the detection zone and/or the control zone.

The labeled receptor is capable of binding to the analyte to be detected. The method is capable of detecting the presence of at least one analyte. The method may be capable of detecting the presence of multiple analytes, such as two or three (different) analytes in a sample.

The labels of the labeled receptor and the labeled control reagent may be different or the same. Visible as well as non-visible labels can be used. Suitable labels include, but are not limited to, fluorescent compounds, chromogenic compounds, chemiluminescent compounds, radioactive compounds, colorimetric compounds, magnetic compounds (*e*.*g*. beads or particles), enzymes, catalytic compounds, substrates, vesicles with labels and particles such as dye particles, colored latex particles, carbon particles, metallic particles, non-metallic particles, colloidal metallic particles. In a preferred embodiment the labels are visible labels with colloidal metallic particles being preferred and gold particles, e.g. colloidal gold particles, being most preferred. The label may be bound to the receptor and/or the control reagent by any suitable means including conjugation, covalent bonding or non-covalent bonding. The label may be directly bound to the receptor and/or the control reagent or the label may be bound through a conjugate such as a biotin-streptavidin conjugate or a biotin-avidin conjugate.

In an embodiment the labeled control reagent is unable to bind to the analyte in the sample. In an embodiment the labeled control reagent forms a specific binding pair with the binding agent immobilized in the control zone of the detection region of the test device. The term 'specific binding pair' as used herein refers to two substances that specifically bind to each other.

In an embodiment the test device is a test strip. In view of the small volumes of liquid sample added to the labeled receptor and the labeled control reagent, it is recommended that the test device is placed such that it rests in the angle between bottom and wall.

In the method of the present invention, when the label density (*i.e.* signal) in the detection zone is higher than that in the control zone, the sample contains no analyte or analyte at a concentration below a given threshold (in other words, the analyte is not present in a sufficient amount and the test is considered 'negative'). In other words, when in the present application reference is made to "the absence of analyte in the sample", it is meant that the sample contains no analyte or analyte at a concentration below a given threshold. However, when the label density in the detection zone is less than the label density in the control zone, analyte is present in the sample at a concentration above a given threshold (in other words, the analyte is present in an amount in excess of allowable levels and the test is considered 'positive'). In other words, when in the present application reference is made to "the presence of analyte in the sample", it is meant that the sample contains analyte at a concentration above a given threshold. When the label density (*i.e.* signal) in the detection zone is equally intense as the label density (*i.e.* signal) in the control zone, analyte is present in the sample at a concentration above, at or below a given threshold. This depends on the chosen threshold. 'Threshold' as used herein refers to the concentration value above which a given analyte is to be regarded as present and below which said analyte is to be regarded as absent. Generally, a threshold value is given for a particular analyte in particular samples by local, regional or interregional authorities, but it can also be pre-set for certain research purposes. The signals may be detected visually by eye, but also by means of a signal reading device such as *e*.*g*. a spectrophotometer, a reflectance reader, a fluorometer, a camera, a magnetic detector, a scintillation counter, to name just a few. An suitable detection device is a smartphone or tablet, preferably a tablet with a holder to position the assay device such that the signals can be read. Such smartphone or tablet may have software capable of analyzing the signals and indicate the test result.

The intensity of the detectable label at the detection zone can be measured to determine the result of the method of the present invention. The method of the present invention may provide a yes or no result (*i.e.* analyte present or absent) or may determine the presence or absence of an analyte above or below a certain threshold value (which is in fact also a yes or no result). The intensity of the signal can be inversely related to the concentration of analyte in the sample. Furthermore, the intensity of the signal at the detection zone can be compared to the intensity of the signal at the control zone to determine a result of the method of the present invention. The difference between the intensities of the various zones can even be analyzed by a signal reading device and used to calculate the concentration of analyte in the sample, for example by comparing the result to a predetermined value.

The section of the device between the proximal and the detection region comprises a compound selected from the list consisting of methylcellulose, carboxymethylcellulose, hydroxymethylcellulose, carboxyethylcellulose, and hydroxyethylcellulose. The inventor has found that methods in the art are often not sensitive enough. He has surprisingly found that the sensitivity towards the analyte can be improved by applying a compound selected from the list consisting of methylcellulose, carboxymethylcellulose, hydroxymethylcellulose, carboxyethylcellulose, and hydroxyethylcellulose to the section of the device between the proximal and the detection region, hereafter referred to as "the compound". In the context of the invention "a compound selected from the list consisting of methylcellulose, carboxymethylcellulose, hydroxymethylcellulose, carboxyethylcellulose, and hydroxyethylcellulose" is understood to include one compound, or two compounds, three compounds, four compounds, or all compounds of the compounds of the list consisting of methylcellulose, carboxymethylcellulose, hydroxymethylcellulose, carboxyethylcellulose, and hydroxyethylcellulose. Thus, the section of the device between the proximal and the detection region may comprise one, two, three, four, or all of the compounds selected from the list consisting of methylcellulose, carboxymethylcellulose, hydroxymethylcellulose, carboxyethylcellulose, and hydroxyethylcellulose.

Methylcellulose, carboxymethylcellulose, hydroxymethylcellulose, carboxyethylcellulose, and hydroxyethylcellulose are commercially available, and their preparation is well known in the art.

In an embodiment the section of the device between the proximal and the detection region does not comprise nitrocellulose and/or cellulose acetate. Thus, in one embodiment the section of the device between the proximal and the detection region does not comprise nitrocellulose. In another embodiment the section of the device between the proximal and the detection region does not comprise cellulose acetate. In yet another embodiment the section of the device between the proximal and the detection region does not comprise either nitrocellulose or cellulose acetate.

The amount of the compound may be between 0.5 and 100 µg. If the section of the device between the proximal and the detection region comprises two or more of the compounds selected from the list consisting of methylcellulose, carboxymethylcellulose, hydroxymethylcellulose, carboxyethylcellulose, and hydroxyethylcellulose, the sum of said two or more compounds may be between 0.5 and 100 µg.

The amount may depend on the length and width of the device. The desired amount can be easily established by the skilled person by varying the amount and recording the sensitivity of the method as a function of the amount.

In an embodiment, the amount of the compound on the section of the device between the proximal and the detection region may be between 1 and 60 µg, more preferably between 2 and 30 µg, more preferably between 3 and 20 µg.

In another embodiment the amount of the compound on the section of the device between the proximal and the detection region may range between 1 and 100 µg/cm², more preferably between 6 and 30 µg/cm².

In another embodiment, the surface area of the section of the device between the proximal and the detection region onto which the compound is applied or is to be applied, is between 0.1 and 2.5 cm², more preferably between 0.2 and 1 cm².

For example, if the surface area of the section of the device between the proximal and the detection region on which the compound is or is to be applied is 0.5 cm², a suitable amount of the compound is between 3 and 12 µg, more preferably between 4 and 10 µg, even more preferably about 6 µg.

The compound may be present anywhere between the proximal end and the detection region of the device. The compound may be evenly distributed on said section, but can also be present on the device in increasing or decreasing amount, starting from the proximal end. The compound may be present continuously or discontinuously. In the latter case, the compound can be present e.g. on two separate regions (with an intermittent region without the compound) or as three regions (with two intermittent regions without the compound), etcetera's. In the context of this invention, 'between the proximal end and the detection region of the device' does not mean that there can be no compound anywhere else on the device, but that there is at least the compound present on the section of the device between the proximal end the detection region. The compound may be on the sample receiving region. It can also be present on the conjugate region. It may also be present in both the sample receiving region and the conjugate region.

In an embodiment the device used in the method of the invention further comprises, between the proximal end and the detection region, a flow-retardation region comprising the compound. This flow-retardation region may be located between the sample receiving section and the conjugate region, or between the conjugate region and the detection region. The device may also comprise two flow-retardation regions, one between the sample receiving section and the other between the conjugate region and the detection region. The compound may also be present on the flow-retardation region(s) and additionally on the sample receiving region and/or the conjugate region.

In an embodiment the analyte is an antibiotic. The term 'antibiotic' as used herein refers to one or more substances or chemical constituents (or metabolites of such substances or chemical compounds) of a sample that display activity against bacteria. In an embodiment of the invention the antibiotic to be detected by the method, test device and/or kit according to the present invention is selected from the group consisting of the family of beta-lactam antibiotics, the family of tetracycline antibiotics, the family of streptomycin antibiotics, the family of sulfonamide antibiotics, the family of aminoglycoside antibiotics, and the family of quinolone antibiotics.

In an embodiment of the invention the analyte to be detected by the method, test device and/or kit according to the present invention is a beta-lactam antibiotic, a tetracycline antibiotic, and/or a streptomycin antibiotic. The method, test device and/or kit according to the present invention may be capable to detect an antibiotic selected from the group consisting of beta-lactam antibiotic, a tetracycline antibiotic, and/or a streptomycin antibiotic. The method, test device and/or kit according to the present invention may be capable to detect a beta-lactam antibiotic and a tetracycline antibiotic. The method, test device and/or kit according to the present invention may be capable to detect a beta-lactam antibiotic and a streptomycin antibiotic. The method, test device and/or kit according to the present invention may also be capable to detect a tetracyclin antibiotic and a streptomycin antibiotic. The method, test device and/or kit according to the present invention may also be capable to detect a beta-lactam antibiotic, a tetracyclin antibiotic, and a streptomycin antibiotic.

The term 'beta-lactam antibiotic' refers to compounds (or metabolites thereof) that comprise a beta-lactam substructure within their chemical structure and display activity against bacteria. Two important subclasses of the beta-lactam antibiotics are the cephalosporin-derived antibiotics and the penicillin-derived antibiotics. Examples of the cephalosporin-derived antibiotics are cefaclor, cefadroxil, cefprozil, ceftiofur, cephalexin, cephapirin and cephradine. Examples of the penicillin-derived antibiotics are amoxicillin, ampicillin, cloxacillin, dicloxacillin, flucloxacillin, oxacillin, penicillin G, penicillin V and ticarcillin.

The term 'tetracycline antibiotic' refers to compounds (or metabolites thereof) that comprise a tetracycline substructure within their chemical structure and display activity against bacteria. Examples of tetracycline antibiotics are tetracycline, oxytetracycline, doxycyclin and chlortetracycline.

The term 'streptomycin antibiotic' refers to streptomycin or dihydrostreptomycin.

The device in the method comprises a conjugate region comprising a labeled receptor capable to bind the analyte and a labeled control reagent.

A suitable receptor to detect a beta-lactam antibiotic is an antibiotic binding protein. Such binding protein may bind a family of antibiotics, which have similar structural binding sites. Suitable binding proteins include, but are not limited to, antibodies (monoclonal, polyclonal or recombinant), antibody fragments, enzymes, aptamers, and receptors such as penicillin binding protein. Preferably, such antibiotic binding protein is a protein obtained from a microorganism. In an embodiment the microorganism is an antibiotic sensitive microorganism. In an embodiment of the invention the organism is selected from the group consisting of a *Bacillus* species, an *Escherichia* species and a *Streptococcus* species. In a preferred embodiment of the invention the organism is thermophilic. Examples are *Bacillus stearothermophilus* or *Streptococcus thermophilus,* with *Bacillus stearothermophilus* being preferred.

A suitable receptor to detect a streptomycin antibiotic is an antibody. The antibody can be either a monoclonal or polyclonal antibody and can be raised in animals such as chicken, goats, guinea pigs, hamsters, mice, sheep etc. Suitable antibodies are also commercially available from several suppliers.

A suitable receptor to detect a tetracyclin antibiotic is an antibody or a binding protein. The antibody can be either monoclonal or polyclonal and can be raised in animals such as chicken, goats, guinea pigs, hamsters, mice, sheep etc. Suitable antibodies are also commercially available from several supplier. The receptor can also be a binding protein such as a natural Tetracycline gene repressor (TetR) protein or mutants thereof. They can be isolated from tetracycline-resistant strains of e.g. E.coli. They can also be produced by cloning into a suitable organism such as E. coli.

The invention also provides a method for detecting a beta-lactam antibiotic, a tetracycline antibiotic, and/or a streptomycin antibiotic in a sample, said method comprising the steps of:
a) providing a test device having a proximal and a distal end, said test device configured to allow lateral flow from the proximal to the distal end, said test device comprising a solid support comprising the following regions in sequence from the proximal to the distal end:
   i. a sample receiving region,
   ii. a conjugate region comprising a labeled antibiotic binding protein, a labeled tetracycline antibody, a labeled streptomycin antibody, and a labeled control reagent,
   iii. a detection region comprising at least two zones:
      A. a first detection zone comprising an immobilized 7-ACA capable of binding the labeled antibiotic binding protein when the labeled antibiotic binding protein is unbound by a beta-lactam antibiotic from the sample,
      B. a second detection zone comprising immobilized tetracyclin capable of binding the tetracycline antibody when the labeled tetracyclin antibody is unbound by a tetracyclin antibiotic from the sample,
      C. a third detection zone comprising immobilized streptomycin, capable of binding the streptomycin antibody when the labeled streptomycin antibody is unbound by a streptomycin antibiotic from the sample, and
      D. a control zone comprising an immobilized binding agent capable of binding the labeled control reagent,
   iv. an absorbing region, and
   v. optionally a handling region,
b) contacting the liquid composition with the sample receiving region of the test device,
c) allowing the liquid composition to move from the sample receiving region through the detection region to the absorbing region, so as to allow the liquid composition comprising the labeled antibiotic binding protein, the-labeled tetracycline antibody, the labeled streptomycin antibody, and the labeled control reagent to contact the detection zone and the control zone,
d) detecting a signal at the detection zone and a signal at the control zone, wherein
   i. the absence of analyte in the sample is indicated by the presence of a signal at the detection zone that is more intense than the signal at the control zone, and
   ii. the presence of analyte in the sample is indicated by the absence of a signal at the detection zone or the presence of a signal at the detection zone that is less intense than the signal at the control zone,
   wherein the section of the device between the proximal end and the detection region comprises a compound selected from the list consisting of methylcellulose, carboxymethylcellulose, hydroxymethylcellulose, carboxyethylcellulose, and hydroxyl-ethylcellulose.

In a second aspect, the invention further relates to a test device for detecting an analyte in a liquid composition, said device having a proximal and a distal end, said test device configured to allow lateral flow from the proximal to the distal end, said test device comprising a solid support comprising the following regions in sequence from the proximal to the distal end:
i. a sample receiving region,
ii. a conjugate region comprising a receptor capable to bind the analyte,
iii. a detection region comprising at least two zones:
   A. a detection zone comprising an immobilized binding agent capable of binding the labeled receptor, when said labeled receptor is unbound by analyte from the sample, and
   B. a control zone comprising an immobilized binding agent capable of binding the labeled control reagent,
iv. an absorbing region, and
v. optionally a handling region,
wherein the section of the device between the proximal and the detection region comprises a compound selected from the list consisting of methylcellulose, carboxymethylcellulose, hydroxymethylcellulose, carboxyethylcellulose, and hydroxyethylcellulose.

'Solid support' as used herein refers to material that is used to provide support for the various regions of the test device. When used for a test device according to the present invention, a solid support usually is made from material that is inert with respect to the application for which the test device is to be used. Suitable materials are glass, metals, and various types of plastics such as for instance polystyrene. The solid support may have a thickness of between 0.1 and 1 mm. In an embodiment the test device may be housed within a non-absorbent or laminate casing. In other words, the test device includes a housing defining an elongated cavity for receiving and holding the test device. Suitable housings are known to a person skilled in the art.

Attachment of the regions to the backing can be performed following known techniques such as gluing, thermo compression and the like.

The test device of the present invention usually has a length varying between 10 and 200 mm, preferably between 20 and 150 mm, more preferably between 30 and 100 mm, and in particular between 50 and 75 mm, a width varying between 1 and 20 mm, preferably between 2 and 15 mm, more preferably between 3 and 10 mm, and a thickness varying between 0.05 and 2 mm, preferably between 0.075 and 1.5 mm, more preferably between 0.1 and 1 mm.

The test device of the present invention has a shelf-life of at least 6 months, preferably of at least 9 months when stored at 4°C. In another embodiment, the test device of the present invention has a shelf-life of up to 10 days, preferably up to 20 days and more preferably up to 28 days when stored at -20°C. In yet another embodiment, the test devices of the present invention have a shelf-life of up to 1 day, preferably up to 4 days and more preferably up to 7 days when stored at 30°C. "Shelf-life" as used herein means that the sensitivity of the stored test device does not decrease. In other words, the sensitivity of a stored test device is equal to the sensitivity of a freshly prepared test device.

In an embodiment the test device can be stored at a temperature of between -20°C and 30°C. Preferably, the test device is stored at a temperature of between 4°C and 8°C.

'Sample receiving region' as used herein refers to the portion of the test device which is brought into direct contact with the liquid composition. The sample receiving section is made of porous material. In a preferred embodiment the sample receiving region is made of a material having a pore size of 3-8 µm. Preferably, the sample receiving region is a polyvinyl alcohol-bound glass fibre membrane such as a Whatman VF2 membrane.

'Conjugate region' as used herein refers to the portion of the test device which is in lateral flow contact with the sample receiving region and with the detection region. The contact can be an end to end connection, but preferably there is overlap between the sample receiving region and the conjugate region and/or between the conjugate region and the detection region. The conjugate region may be made of porous material e.g. glass (micro)fiber, polyethylene and polyester.

'Detection region' as used herein refers to the portion of the test device, which is in lateral flow contact with the conjugate region and the absorbing region. The contact can be an end-to-end connection, but preferably there is overlap between the detection region and the conjugate region and between the detection region and the absorbing region. The overlap may be between 1 and 2 mm. The detection region is made of porous material. The detection region comprises at least a detection zone for detecting the presence or absence of the antibiotic and a control zone that functions as a control site. The detection region may have two or more detection zones and/or two or more control zones. The detection zones may have the same functionality or may have different functionalities (*i.e.* receptors at the different detection zones may be capable of binding to the same compound or may be capable of binding to different compounds). The same holds true for the control zones. The one or more zones may be made of a porous material different than that of the detection region. The separate zones may be made of a different porous material. Preferably, they are made of the same material. Preferably, the one or more zones are made of the same material as the detection region. When the liquid composition moves through the detection region, it may first be contacted with the detection zone and then be contacted with the control zone or *vice versa.* If the detection region has several detection zones and/or control zones, any sequence of zones may be provided. The zones can be in a variety of configurations including lines, dots or other configurations. In a preferred embodiment the zones are lines.

'Lateral flow' as used herein refers to liquid flow of a sample in a material in which all of the dissolved and/or dispersed components of the sample are transported at essentially equal velocities and with relatively unimpaired flow laterally through the material.

The detection zone and the control zone each may comprise at least one immobilized binding agent. Preferably, the detection zone and control zone are made by applying the appropriate binding agents or mixture of binding agents to the detection region, either by means of covalent linkages or other bonding processes. 'Binding agent' as used herein refers to any reagent that can be used to create the required functionality in the detection and/or control zone. The application (immobilization) of the binding agent to the detection region can be done by known methods such as spraying, dispensing, painting, drawing, printing, striping and the like. The zones are capable of generating a signal, for instance a visual color signal, upon presence or absence of the complex between the binding agent and its binding partner. In the art, a 'binding agent' is sometimes also referred to as 'receptor' (not to be confused with *labeled* receptor). Likewise, "immobilized binding agent" may also be referred to as "immobilized analyte".

A binding agent may be any natural or non-natural compound. Examples of suitable binding agents are antibiotics, antibodies, antigens, ligands, proteins, to name just a few. The binding agent (capable of binding the labeled receptor) at the detection zone of the test device according to the present invention may be an antibiotic or an analogue thereof, which may be immobilized at the detection zone at a concentration of 1-3 mg/mm. Preferably, the antibiotic or analogue thereof is present in a Tris buffer when immobilized to the detection zone. Examples of suitable antibiotics or analogues thereof are beta-lactam antibiotics, *e*.*g*. cephalosporins such as for instance 7-amino-cephalosporanic acid (7ACA), streptomycin, and tetracyclin. Preferably, an antibiotic or analogue thereof is immobilized on the test device and is capable of binding to the labeled receptor when said labeled receptor is unbound by antibiotic from the sample. When the labeled receptor is bound by antibiotic from the sample, the immobilized antibiotic will not be able to bind to the labeled receptor.

In an embodiment the binding agent (capable of binding the labeled control reagent) at the control zone of the test device according to the present invention is a member of a binding pair *e*.*g*. a specific binding pair such as an antigen/antibody pair. It may however also be an antibody binding protein such as *e*.*g*. protein A. The binding agent (capable of binding the labeled control reagent) may be present in a solution comprising an additional protein *e.g.* bovine serum albumin, a sugar *e.g.* a disaccharide such as saccharose and a salt such as NaCl when applied to the control zone. In an embodiment the binding agent (capable of binding the labeled control reagent) at the control zone is unable to bind to the analyte in the sample and/or the labeled receptor, irrespective of whether the analyte receptor has analyte bound or not.

Immobilization of the binding agent to the detection region may be carried out in a manner known in the art, for example by covalent or non-covalent adsorption to the detection region. The binding agent may also be covalently conjugated to the detection region through a carrier such as for instance bovine serum albumin (BSA). Optionally, the binding agent may be coupled to the carrier via a spacer. Many bifunctional compounds are suitable as a spacer. All methods available to construct bonds, *e*.*g*. coupling techniques for instance those known from peptide chemistry, could be applied, unless they are detrimental to the binding agent. Suitable spacers, carriers and coupling techniques are well known to the person skilled in the art.

The control zone produces a signal irrespective of whether or not an analyte is present in the sample and gives an indication that the test device functions as required. The control zone provides a consistent signal that does not vary with the concentration of analyte in the sample. The control zone can also be used to inform the user that the liquid composition has flowed through the test device. In that sense, the control zone can be used as a flow control. Furthermore, the control zone can be used for comparison to the detection zone.

'Absorbing region' as used herein refers to the part of the test device, which is in lateral flow contact with the detection region and functions to promote lateral flow through the detection region and is capable of absorbing excess liquid sample. The contact an end-to-end connection, but preferably is an overlap between the detection region and the absorbing region. The overlap may be between 1 and 2 mm. The absorbing region is made of porous material. In a preferred embodiment the absorbing region is at least 1 cm.

'Handling region' as used herein relates to a region of the test device that can be used to hold and manipulate the test device without interfering with the test result. The handling region may be a separate region attached to the solid support, but the handling region can also be a part of the solid support itself. The handling region and absorbing region may also be combined into one region, if desired.

In yet another embodiment of the present invention, the test device comprises a member that covers one or more of the sample receiving region, the detection region and the absorbing region. Said member, which can be made of any material, preferably a clear plastic material, advantageously provides protection for said regions with regard to fingerprints and/or mechanical destruction and/or fumes and the like. One or more regions may be covered with a single member, however also multiple members optionally of different materials may be used.

'Porous material' as used herein refers to any material capable of providing lateral flow. Examples of suitable porous materials are polymeric materials such as polyvinyl or polyester, cotton, glass fiber, nitrocellulose, blends of nitrocellulose with polymeric materials, nylon, paper, rayon and the like.

In an embodiment the test device comprises a detection region that is longer than the absorbing region, an absorbing region that is longer than the handling region, and a handling region that is longer than the sample receiving region.

The test devices according to the present invention are manufactured by the methods known to the skilled in the art. Solid supports can have the form of cards. These can be prepared, for example, using commercially available laminators. The cards can be laminated. Onto the cards the various regions can be attached. The binding agent is deposited on the detection region in the form of a solution, before or after the assembly of the cards. These solutions can be deposited very precisely using commercially available apparatus such as dispensers from BioDot, Inc. Before and/or after application of the detection and/or control zone, the detection region can be blocked by for instance spraying a blocking solution. Preferred blocking solutions comprise a buffer *e*.*g*. an organic buffer such as a Tris buffer, a surfactant *e*.*g*. Tween such as Tween-20, and a protein such as bovine serum albumin. Preferably, the blocking solution is not sprayed directly onto the detection zone and/or control zone. The deposited solutions can be immediately evaporated, for example, by placing the card under a stream of hot air. After all regions and zones have been applied, the cards are preferably dried in a dry atmosphere, *i.e.* an atmosphere having a relative humidity of < 50%, < 40%, < 30%, < 20%, < 10%, preferably 0%. For large-scale production it is also possible to prepare rolls. Subsequently, the cards and rolls bearing the desired receptor can be cut into strips, each of these strips constituting a test device according to the invention. Alternatively, the binding agent can also be deposited on the detection region prior to the assembly of the cards or rolls by simply immersing the detection region in a solution containing the binding agent.

The methylcellulose, carboxymethylcellulose, hydroxymethylcellulose, carboxyethylcellulose, and/or hydroxyethylcellulose can be applied to the device by applying an aqueous suspension to the device, e.g. by dripping or spraying. The methylcellulose, carboxymethylcellulose, hydroxymethylcellulose, carboxyethylcellulose, and/or hydroxyethylcellulose can be applied to the device together with the labeled receptor.

In a third aspect, the current invention is also directed to a kit comprising a container to hold the sample and a test device according to the present invention. The container may contain compounds such as a buffer such as a phosphate buffer and a surfactant such as Tween 20.

The test device is preferably stored in a package comprising a desiccant. Preferably, the kit comprises more than one container and more than one test device, e.g. 10, 20, 30, 40, 50 or even 100 containers and/or test devices. The kit according to the present invention may also comprise a sampling device. This is a device with the aid of which sample, *e*.*g*. liquid sample, can be added to the container. Examples include, but are not limited to, a container (optionally with volume markings), a syringe, a pipette or an automated pipetting system. Such a syringe or pipette may be designed in such a fashion that with only one mode of operation a predetermined volume can be withdrawn from the liquid sample to be analyzed. Optionally, systems known in the art with which more than one syringe or pipette can be operated with one single handling may be applied. In case, the kit comprises a sampling device such as a pipette, it may additionally comprise pipette tips. Preferably, the amount of pipette tips is equal to the amount of containers (*i.e.* the containers wherein the labeled receptor and labeled control reagent are present) and test devices. This way, with only one pipette different samples can be applied to different containers. If the kit comprises disposable pipettes as sampling device, the amount of disposable pipettes is equal to the amount of containers (*i.e.* the containers wherein the labeled receptor and labeled control reagent are present) and test devices.

Optionally, the kit further comprises an insert with instructions for use and/or a means for setting the time needed for incubation. Optionally, the kit further comprises a thermostatic device such as an incubator or a water bath, with the aid of which samples can be kept at a pre-set temperature, such as the temperature at which the liquid sample, the labeled receptor, the labeled control reagent and optionally the test device should be incubated. Preferably, said thermostatic device is designed in such a fashion that it can hold the containers filled with the labeled receptor, the labeled control reagent, the liquid sample and optionally the test device. Optionally, the thermostatic device is coupled to a means for setting the time needed for incubation such that heating is stopped after lapse of a pre-set period. Optionally, the kit also comprises a sample-reading device, a data carrier loaded with a computer program suitable for instructing a computer to analyze digital data obtained from the sample-reading device.

The embodiments and features disclosed above for the method of the present invention also pertain to the test device and kit according to the present invention. The embodiments and features disclosed above for the test device of the present invention also pertain to the method and kit according to the present invention. The embodiments and features disclosed above for the kit of the present invention also pertain to the test device and method according to the present invention.

### EXAMPLES

### Example 1

### Preparation of the labeled antibiotic binding protein and labeled control reagent

Streptavidin-coated gold particles were synthesized by reacting colloidal gold (diameter 40 nm; 1 OD/ml) with 10 µg/ml streptavidin. Next, the obtained solution was concentrated by tangential flow filtration, washed and stored in a Tris buffer (pH 8) including NaN₃. Penicillin binding protein purified from *Bacillus stearothermophilus* was biotinylated (1:4) with D-biotinyl-ε-aminocaproic acid-N-hydroxysuccinimide ester. Next, the penicillin binding protein-gold conjugate was synthesized by reacting 1.5 µg biotinylated penicillin binding protein with 1 OD streptavidin-coated gold particles for 1 hour, followed by centrifugation at 10,000xg for 5 minutes and resuspension of the obtained pellet in 45 mM bicarbonate buffer comprising 0.1 % w/v Triton X-100 (pH 9.6).

IgY-coated gold particles were synthesized by reacting colloidal gold (diameter 40 nm; 1 OD/ml) with 10 µg/ml IgY. Next, the obtained solution was centrifuged at 7,500xg for 10 minutes and the obtained pellet was resuspended in a Tris buffer (pH 8), washed by another round of centrifugation and resuspension of the obtained pellet in a Tris buffer (pH 8) and stored in a Tris-buffer (pH 8) containing NaN₃.

### Preparation of the test device

### Detection and control regions

A nitrocellulose membrane (Sartorius CN95; length 42 mm) was used as a detection region. The membrane was glued to a polystyrene laminated card 17 mm from the proximal end of the card.

The detection zone and control zone were applied onto the nitrocellulose membrane. The detection zone was applied by dispensing 1 mg/ml of a 7ACA-spacer-IgG conjugate in 20 mM KPO4-buffer (pH 7.5) with a Biodot Dispense workstation XYZ 3050 with frontline at 0.2 µl/cm (position 31 mm from proximal end). The control zone was applied by dispensing 0.15 mg/ml of an anti-lgY antibody in 20 mM KPO₄- buffer (pH 7.5) comprising 0.675 mg/ml BSA, 5% w/v saccharose and 20 mM NaCl with a Biodot Dispense workstation XYZ 3050 with frontline at 0.8 µl/cm (position 36 mm from the proximal end of the card). The obtained cards were dried at 37°C in a dry atmosphere (0% relative humidity).

### Conjugate pads

The conjugate pad (Porex PE, length 0.85 cm) was prepared by applying a suspension containing the gold-labeled antibiotic binding protein and gold-labeled control reagent (see example 1), 40 mM Tris pH 7.4, 10% Sucrose, 1 mgmL⁻¹ BSA, 1% Tween-20 and Hydroxyethylcellulose (HEC). Three variants were produced with suspensions containing 0 (variant 1), 0.25 (variant 2) or 0.5 % HEC (variant 3). The suspension way sprayed onto the conjugate pad using a Biodot Dispense workstation XYZ 3050 with an airjet operated at 5 µL/cm. The obtained conjugate pads were dried at ambient temperature in a dry atmosphere (0% relative humidity).

### Assembly

The conjugate pad was glued onto the polystyrene laminated card 8.5 mm from the proximal end of the card, overlapping 2 mm with the nitrocellulose membrane. The sample receiving region (Millipore VF2, length 8.5 mm) was glued at the proximal end of the card overlapping 2 mm with the conjugate pad.

The absorbing region (10038 membrane from Millipore; length 18.5 mm) was glued at the distal end of the card with an overlap of 2 mm on the nitrocellulose membrane. The card was cut in strips of 5.4 mm width and kept at a dry atmosphere (0% relative humidity).

The amount of the HEC in the 3 variants mentioned above are respectively 0, 6.6 and 13.2 µg per test device.

### Detection of antibiotics with the test device

150 µl of milk sample (antibiotic-free or PenG-spiked) was added in a tube containing a dried buffer (end concentration: 1% Tween 20, 50 mM KPO₄-Buffer pH 8) and mixed for 30 seconds. The concentration of penicillin G in the added milk was 0 or 4 ng/g. The tube with the obtained liquid composition was placed in a heating block at 64°C, the test device was added to the tube and incubated for 7 minutes at 64°C. After incubation, the test device was removed from the tube and read using a reader (Qiagen ESEQuant lateral flow reader). The results are shown in the table below.

| Variant | HEC in conjugate pad [µg] | Test line signal with sample containing 0 ppb PenG [mV] | Test line signal with sample containing4 ppb PenG [mV] | Reduction of Test line signal by the presence of 4 ppb PenG |
|---|---|---|---|---|
| Variant 1 | 0 | 475 | 321 | 32% |
| Variant 2 | 6.6 | 527 | 306 | 42% |
| Variant 3 | 13.2 | 478 | 266 | 44% |

The results show that applying Hydroxyethylcellulose to the conjugate pad improves the sensitivity of the test device. The device with conjugate pads containing 0.25 and 0.50% HEC show increasingly higher signal reduction by the presence of 4 ppb PenG in the test sample.

## Claims

1. A method for detecting an analyte in a liquid composition, said method comprising:
a) providing a test device having a proximal and a distal end, said test device configured to allow lateral flow from the proximal to the distal end, said test device comprising a solid support comprising the following regions in sequence from the proximal to the distal end:
i. a sample receiving region,
ii. a conjugate region comprising a labeled receptor capable to bind the analyte and a labeled control reagent,
iii. a detection region comprising at least two zones:
A. a detection zone comprising an immobilized binding agent capable of binding the labeled receptor, when said labeled receptor is unbound by analyte from the sample, and
B. a control zone comprising an immobilized binding agent capable of binding the labeled control reagent,
iv. an absorbing region,
b) contacting the liquid composition with the sample receiving region of the test device,
c) allowing said liquid composition to move from the sample receiving region through the conjugate region and the detection region to the absorbing region, so as to allow said liquid composition comprising the labeled receptor and the labeled control reagent to contact the detection zone and the control zone,
d) detecting a signal at the detection zone and a signal at the control zone, wherein
i. the absence of analyte in the sample is indicated by the presence of a signal at the detection zone that is more intense than the signal at the control zone, and
ii. the presence of analyte in the sample is indicated by the absence of a signal at the detection zone or the presence of a signal at the detection zone that is less intense than the signal at the control zone,
wherein the section of the device between the proximal and the detection region comprises a compound selected from the list consisting of methylcellulose, carboxymethylcellulose, hydroxymethylcellulose, carboxyethylcellulose, and hydroxyethylcellulose.

2. Method according to claim 1 wherein the section of the device between the proximal and the detection region does not comprise nitrocellulose and/or cellulose acetate.

3. Method according to claim 1 or 2 wherein the analyte is a beta-lactam antibiotic, a tetracyclin antibiotic, and/or a streptomycin antibiotic.

4. Method according to any of claim 1-3 wherein the amount of the compound selected from the list consisting of methylcellulose, carboxymethylcellulose, hydroxymethylcellulose, carboxyethylcellulose, and hydroxyethylcellulose is between 0.5 and 100 µg.

5. Method according to any one of the claims 1 to 4 wherein the device further comprises, between the proximal end and the detection region, a flow-retardation region comprising said compound selected from the list consisting of methylcellulose, carboxymethylcellulose, hydroxymethylcellulose, carboxyethylcellulose, and hydroxyethylcellulose.

6. Method according to claim 5 wherein the compound selected from the list consisting of methylcellulose, carboxymethylcellulose, hydroxymethylcellulose, carboxyethylcellulose, and hydroxyethylcellulose is present on the sample receiving region and/or the conjugate region and/or the flow-retardation region.

7. Method according to any one of the claims 1 to 6 wherein the test device is a test strip.

8. Method according to any one of the claims 1 to 7 wherein the immobilized binding agent is bound to the solid support by a spacer-protein conjugate.

9. Method according to any one of the claims 1 to 8, wherein the test device is contacted with the liquid composition for 1 to 10 minutes at a temperature of 40 to 70°C.

10. Method according to any one of the claims 1 to 9 wherein the liquid composition is milk.

11. Method according to any one of the claims 1 to 10 wherein the volume of the liquid composition that is contacted with the sample receiving region is between 50 and 500 µl.

12. A test device for detecting an analyte in a liquid composition, said device having a proximal and a distal end, said test device configured to allow lateral flow from the proximal to the distal end, said test device comprising a solid support comprising the following regions in sequence from the proximal to the distal end:
i. a sample receiving region,
ii. a conjugate region comprising a labeled receptor capable to bind the analyte,
iii. a detection region comprising at least two zones:
A. a detection zone comprising an immobilized binding agent capable of binding the labeled receptor, when said labeled receptor is unbound by analyte from the sample, and
B. a control zone comprising an immobilized binding agent capable of binding the labeled control reagent,
iv. an absorbing region,
wherein the section of the device between the proximal end and the detection region comprises a compound selected from the list consisting of methylcellulose, carboxymethylcellulose, hydroxymethylcellulose, carboxyethylcellulose, and hydroxyethylcellulose.

13. A kit comprising a container comprising a buffer, and a test device according to claim 12.

14. A kit according to claim 13, further comprising a pipette and/or a manual.

## Patentansprüche

1. Verfahren zum Nachweis eines Analyten in einer flüssigen Zusammensetzung, wobei das Verfahren Folgendes umfasst:
a) die Bereitstellung eines Testgeräts mit einem proximalen und einem distalen Ende, wobei das Testgerät so konfiguriert ist, dass es einen lateralen Durchfluss vom proximalen zum distalen Ende erlaubt, wobei das Testgerät einen festen Träger umfasst, der in Reihenfolge vom proximalen zum distalen Ende die folgenden Regionen umfasst:
i. eine Probenaufnahmeregion,
ii. eine Konjugatregion, die einen zur Bindung des Analyten fähigen markierten Rezeptor und ein markiertes Kontrollreagenz umfasst,
iii. eine Nachweisregion, die mindestens zwei Zonen umfasst:
A. eine Nachweiszone, die ein immobilisiertes Bindungsmittel umfasst, das zur Bindung des markierten Rezeptors fähig ist, wenn der markierte Rezeptor nicht durch den Analyten aus der Probe gebunden ist, und
B. eine Kontrollzone, die ein immobilisiertes Bindungsmittel umfasst, das zur Bindung des markierten Kontrollreagenz fähig ist,
iv. eine absorbierende Region,
b) das Inkontaktbringen der flüssigen Zusammensetzung mit der Probenaufnahmeregion des Testgeräts,
c) das Ermöglichen des Strömens der flüssigen Zusammensetzung von der Probenaufnahmeregion durch die Konjugatregion und die Nachweisregion zur absorbierenden Region, so dass es der den markierten Rezeptor und das markierte Kontrollreagenz umfassenden flüssigen Zusammensetzung möglich ist, mit der Nachweiszone und der Kontrollzone in Kontakt zu treten,
d) die Detektion eines Signals in der Nachweiszone und eines Signals in der Kontrollzone, wobei
i. die Abwesenheit des Analyten in der Probe durch das Vorhandensein eines Signals in der Nachweiszone, das intensiver ist als das Signal in der Kontrollzone, angezeigt wird und
ii. das Vorhandensein des Analyten in der Probe durch die Abwesenheit eines Signals in der Nachweiszone oder das Vorhandensein eines Signals in der Nachweiszone, das weniger intensiv ist als das Signal in der Kontrollzone, angezeigt wird,
wobei der Abschnitt des Geräts zwischen der proximalen Region und der Nachweisregion eine aus der aus Methylcellulose, Carboxymethylcellulose, Hydroxymethylcellulose, Carboxyethylcellulose und Hydroxyethylcellulose bestehenden Liste ausgewählte Verbindung umfasst.

2. Verfahren nach Anspruch 1, wobei der Abschnitt des Geräts zwischen der proximalen Region und der Nachweisregion keine Nitrocellulose und/oder Celluloseacetat umfasst.

3. Verfahren nach Anspruch 1 oder 2, wobei es sich bei dem Analyten um ein beta-Lactam-Antibiotikum, ein Tetracyclinantibiotikum und/oder ein Streptomycinantibiotikum handelt.

4. Verfahren nach einem der Ansprüche 1-3, wobei die Menge der aus der aus Methylcellulose, Carboxymethylcellulose, Hydroxymethylcellulose, Carboxyethylcellulose und Hydroxyethylcellulose bestehenden Liste ausgewählten Verbindung zwichen 0,5 und 100 µg liegt.

5. Verfahren nach einem der Ansprüche 1 bis 4, wobei das Gerät weiterhin zwischen dem proximalen Ende und der Nachweisregion eine strömungsverlangsamende Region umfasst, die die aus der aus Methylcellulose, Carboxymethylcellulose, Hydroxymethylcellulose, Carboxyethylcellulose und Hydroxyethylcellulose bestehenden Liste ausgewählte Verbindung umfasst.

6. Verfahren nach Anspruch 5, wobei die aus der Methylcellulose, Carboxymethylcellulose, Hydroxymethylcellulose, Carboxyethylcellulose und Hydroxyethylcellulose bestehenden Liste ausgewählte Verbindung in der Probenaufnahmeregion und/oder der Konjugatregion und/oder der strömungsverlangsamenden Region vorhanden ist.

7. Verfahren nach einem der Ansprüche 1 bis 6, wobei es sich bei dem Testgerät um einen Teststreifen handelt.

8. Verfahren nach einem der Ansprüche 1 bis 7, wobei das immobilisierte Bindungsmittel über ein Spacer-Protein-Konjugat an den festen Träger gebunden ist.

9. Verfahren nach einem der Ansprüche 1 bis 8, wobei man das Testgerät bei einer Temperatur von 40 bis 70°C 1 bis 10 Minuten mit der flüssigen Zusammensetzung in Kontakt bringt.

10. Verfahren nach einem der Ansprüche 1 bis 9, wobei es sich bei der flüssigen Zusammensetzung um Milch handelt.

11. Verfahren nach einem der Ansprüche 1 bis 10, wobei das Volumen der flüssigen Zusammensetzung, das mit der Probenaufnahmeregion in Kontakt gebracht wird, zwischen 50 und 500 µl liegt.

12. Testgerät zum Nachweis eines Analyten in einer flüssigen Zusammensetzung, wobei das Testgerät ein proximales und ein distales Ende aufweist, wobei das Testgerät so konfiguriert ist, dass es einen lateralen Durchfluss vom proximalen zum distalen Ende erlaubt, wobei das Testgerät einen festen Träger umfasst, der in Reihenfolge vom proximalen zum distalen Ende die folgenden Regionen umfasst:
i. eine Probenaufnahmeregion,
ii. eine Konjugatregion, die einen zur Bindung des Analyten fähigen markierten Rezeptor umfasst,
iii. eine Nachweisregion, die mindestens zwei Zonen umfasst:
A. eine Nachweiszone, die ein immobilisiertes Bindungsmittel umfasst, das zur Bindung des markierten Rezeptors fähig ist, wenn der markierte Rezeptor nicht durch den Analyten aus der Probe gebunden ist, und
B. eine Kontrollzone, die ein immobilisiertes Bindungsmittel umfasst, das zur Bindung des markierten Kontrollreagenz fähig ist,
iv. eine absorbierende Region,
wobei der Abschnitt des Geräts zwischen der proximalen Region und der Nachweisregion eine aus der aus Methylcellulose, Carboxymethylcellulose, Hydroxymethylcellulose, Carboxyethylcellulose und Hydroxyethylcellulose bestehenden Liste ausgewählte Verbindung umfasst.

13. Kit, umfassend einen einen Puffer umfassenden Behälter und ein Testgerät nach Anspruch 12.

14. Kit nach Anspruch 13, weiterhin umfassend eine Pipette und/oder ein Handbuch.

## Revendications

1. Procédé de détection d'un analyte dans une composition liquide, ledit procédé comprenant :
a) la fourniture d'un dispositif d'essai ayant une extrémité proximale et une extrémité distale, ledit dispositif d'essai étant configuré pour permettre un écoulement latéral de l'extrémité proximale vers l'extrémité distale, ledit dispositif d'essai comprenant un support solide comprenant les régions suivantes en séquence de l'extrémité proximale à l'extrémité distale :
i. une région de réception d'échantillon,
ii. une région conjuguée comprenant un récepteur marqué pouvant se lier à l'analyte et un réactif de contrôle marqué,
iii. une région de détection comprenant au moins deux zones :
A. une zone de détection comprenant un agent de liaison immobilisé pouvant se lier au récepteur marqué, lorsque ledit récepteur marqué n'est pas lié par l'analyte provenant de l'échantillon, et
B. une zone de contrôle comprenant un agent de liaison immobilisé pouvant se lier au réactif de contrôle marqué,
iv. une région d'absorption,
b) la mise en contact de la composition liquide avec la région de réception d'échantillon du dispositif d'essai,
c) le déplacement de ladite composition liquide depuis la région de réception d'échantillon à travers la région conjuguée et la région de détection vers la région d'absorption, de façon à permettre que ladite composition liquide comprenant le récepteur marqué et le réactif de contrôle marqué entre en contact avec la zone de détection et la zone de contrôle,
d) la détection d'un signal au niveau de la zone de détection et d'un signal au niveau de la zone de contrôle, dans lequel
i. l'absence d'analyte dans l'échantillon est indiquée par la présence d'un signal au niveau de la zone de détection qui est plus intense que le signal au niveau de la zone de contrôle, et
ii. la présence d'analyte dans l'échantillon est indiquée par l'absence d'un signal au niveau de la zone de détection ou la présence d'un signal au niveau de la zone de détection qui est moins intense que le signal au niveau de la zone de contrôle,
dans lequel la section du dispositif entre la région proximale et la région de détection comprend un composé choisi dans la liste constituée de la méthylcellulose, la carboxyméthylcellulose, l'hydroxyméthylcellulose, la carboxyéthylcellulose et l'hydroxyéthylcellulose.

2. Procédé selon la revendication 1, dans lequel la section du dispositif entre la région proximale et la région de détection ne comprend pas de nitrocellulose et/ou d'acétate de cellulose.

3. Procédé selon la revendication 1 ou 2, dans lequel l'analyte est un antibiotique bêta-lactamine, un antibiotique tétracycline et/ou un antibiotique streptomycine.

4. Procédé selon l'une quelconque des revendications 1 à 3, dans lequel la quantité du composé choisi dans la liste constituée de la méthylcellulose, la carboxyméthylcellulose, l'hydroxyméthylcellulose, la carboxyéthylcellulose et l'hydroxyéthylcellulose est comprise entre 0,5 et 100 µg.

5. Procédé selon l'une quelconque des revendications 1 à 4, dans lequel le dispositif comprend en outre, entre l'extrémité proximale et la région de détection, une région de retard d'écoulement comprenant ledit composé choisi dans la liste constituée de la méthylcellulose, la carboxyméthylcellulose, l'hydroxyméthylcellulose, la carboxyéthylcellulose et l'hydroxyéthylcellulose.

6. Procédé selon la revendication 5 dans lequel le composé choisi dans la liste constituée de la méthylcellulose, la carboxyméthylcellulose, l'hydroxyméthylcellulose, la carboxyéthylcellulose et l'hydroxyéthylcellulose est présent sur la région de réception d'échantillon et/ou la région conjuguée et/ou la région de retard d'écoulement.

7. Procédé selon l'une quelconque des revendications 1 à 6, dans lequel le dispositif d'essai est une bandelette réactive.

8. Procédé selon l'une quelconque des revendications 1 à 7, dans lequel l'agent de liaison immobilisé est lié au support solide par un conjugué espaceur-protéine.

9. Procédé selon l'une quelconque des revendications 1 à 8, dans lequel le dispositif d'essai est mis en contact avec la composition liquide pendant 1 à 10 minutes à une température de 40 à 70 °C.

10. Procédé selon l'une quelconque des revendications 1 à 9, dans lequel la composition liquide est du lait.

11. Procédé selon l'une quelconque des revendications 1 à 10, dans lequel le volume de la composition liquide qui est mis en contact avec la région de réception d'échantillon est compris entre 50 et 500 µl.

12. Dispositif d'essai pour détecter un analyte dans une composition liquide, ledit dispositif ayant une extrémité proximale et une extrémité distale, ledit dispositif d'essai étant configuré pour permettre un écoulement latéral de l'extrémité proximale vers l'extrémité distale, ledit dispositif d'essai comprenant un support solide comprenant les régions suivantes en séquence de l'extrémité proximale à l'extrémité distale :
i. une région de réception d'échantillon,
ii. une région conjuguée comprenant un récepteur marqué pouvant se lier à l'analyte,
iii. une région de détection comprenant au moins deux zones :
A. une zone de détection comprenant un agent de liaison immobilisé pouvant se lier au récepteur marqué, lorsque ledit récepteur marqué n'est pas lié par l'analyte provenant de l'échantillon, et
B. une zone de contrôle comprenant un agent de liaison immobilisé pouvant se lier au réactif de contrôle marqué,
iv. une région d'absorption,
dans lequel la section du dispositif entre l'extrémité proximale et la région de détection comprend un composé choisi dans la liste constituée de la méthylcellulose, la carboxyméthylcellulose, l'hydroxyméthylcellulose, la carboxyéthylcellulose et l'hydroxyéthylcellulose.

13. Trousse comprenant un récipient comprenant un tampon, et un dispositif d'essai selon la revendication 12.

14. Trousse selon la revendication 13, comprenant en outre une pipette et/ou un manuel.
